# EUROPEAN PATENT APPLICATION

(11) **EP 1 413 876 A2**
(43) Date of publication of application: **28.04.2004**
(21) Application number: 03256746.3
(22) Date of filing: 24.10.2003
(51) Int. Cl.: G01N 21/41, G01N 21/55, G01N 33/48

(54) **Multi-mode planar waveguide sensor, fabrication materials and techniques**

(30) Priority: 25.10.2002 US 280188
(71) Applicant: General Dynamics Advanced Information Systems, Inc., Ann Arbor, Michigan 48105 (US)
(72) Inventor: Datesman, Aaron M., Charlottesville, Virginia 22901 (US); Barker, Susan L.R., Charlottesville, Virginia 22901 (US); Wagner,Michael L., Ruckersville, Virginia 22968 (US); Angeley, David, Charlottesville, Virginia 22911 (US); Black, Eric, Charlottesville, Virginia 22901 (US)
(74) Representative: Naismith, Robert Stewart

(57) **Abstract**

A simple, label-less, sensitive, rapid, and inexpensive sensor incorporates a unique optical transduction design and novel materials optimized for chemical or biosensing. The sensor features a planar coupler with at least one input and two output optical waveguides. The system responds to changes in refractive index upon analyte binding to molecular receptors on the sensor surface. In the preferred embodiment the coupling region can support at least two electromagnetic modes, and the interaction of these fields determines the amount of light propagating down each of the output waveguides. The changes in refractive index at the surface of the coupled region affect the interaction of the two propagation modes and, therefore, the amount of light detected at each output. In addition to a unique mode of operation, the waveguides and coupler are preferably fabricated from sol-gel materials chosen to optimize physical properties, suitability for lithography, optical properties, and availability of functional groups for immobilization of receptors or molecular recognition elements. The sol-gel materials used can be modified with dopants, allowing facile tailoring of their refractive index. Refractive index tuning is an advantage, not only for optimization of the coupler's waveguiding properties and refractive index sensitivity, but also allows the sensor to be tuned to have highest sensitivity in the refractive index ranges necessary for detection of protein/oligonucleotide-based molecular-based molecular recognition, or other analytes of interest.

## Description

### Field of the Invention

This invention relates generally to the detection of chemical and biological materials and, in particular, to a sensor involving a signal-transduction platform and novel materials capable of rapid detection, specific identification, and real-time reporting.

### Background of the Invention

Advances in chemical and biological weapons threaten both military and civilian personnel. The ability to sensitively detect infectious diseases is also a critical concern, given the desire to detect pathogens that threaten civilian populations such as *E.coli* 1057:H7, prion proteins causing spongiform-encephalitis (Mad Cow disease), and the West Nile virus.

The current 'gold standard' methodology used to accurately identify a pathogen is the century old technique of "swab and culture." Technicians place bacterial samples in enrichment medium, then grow and identify them using biological assays that measure specific metabolic profiles. Viruses and rickettsia are detected through techniques such as immunoassays that detect viral antigen or polymerase chain reaction (PCR) amplification and fluorescence-based oligonucleotide detection that detects production of virus genes. Unfortunately, these methods require laboratory facilities, expertise in culturing methods, expertise in interpretation of results, and long incubation periods. Therefore, various research efforts have been initiated in to develop rapid, portable sensor systems that can be used in conjunction with or possibly even replace laboratory techniques.

Numerous fluorescence-based sensors have also been developed in a variety of formats, including planar, evanescent sensors. Fluorescence is currently used in antibody-antigen detection, as well as in the detection of nucleic acids (gene chips). Unfortunately, the need for fluorescent tags limits the utility of these sensors. In order to avoid labeling with fluorescent dye molecules, a variety of label-less techniques have been developed that measure changes in refractive index, including surface plasmon resonance (SPR), resonant mirrors, and interferometry. However, in published results, none of these systems have demonstrated protein detection at sensitivities greater than ng/mL (For typical 150 kDa proteins in water 1 ng/mL ≅ 5 pM ≅ 1 ppb), and many involve complicated optical configurations.

Thus, there exists a basic need in the defense, medical, environmental and domestic preparedness communities to accurately detect trace analytes out of a complex background both specifically and rapidly. Although some sensors now exist to detect pathogens that pose risk, current technology lacks the capability to provide unique, sensitive, portable, rapid detection. Due to the low concentration and speed at which pathogens are able to infect, a new generation of chemical and biological sensors are needed.

### Summary of the Invention

Broadly, this invention resides in the category of a simple, label-less, sensitive, rapid, and inexpensive sensor. In the preferred embodiment, the sensor incorporates a unique optical transduction design, as well as novel materials, all of which are optimized for biosensing. The simplified design and fabrication methods make future reproducible mass production a possibility.

The apparatus incorporates a planar coupler with at least one input and two output optical waveguides. The coupling region can support at least two electromagnetic modes, and the interaction of the fields of the modes determines the amount of light propagating down each of the output waveguides. The system responds to changes in refractive index upon analyte binding to receptors on the sensor surface in the vicinity of the coupling region. The changes in refractive index at the surface of the coupled region affect the interaction of the two propagation modes and, therefore, the amount of light detected at each output. The coupler also preferably includes a light intensity reference waveguide as a means to measure and eliminate any remaining bulk index signal from the output signals. To enhance throughput, the couplers will be fabricated in an array format.

In addition to a unique mode of operation compared to planar sensor systems currently in use, the waveguides are preferably fabricated from sol-gel materials. The sol-gel formulations are chosen to optimize physical properties, suitability for lithography, optical properties, and availability of functional groups for immobilization of biological or chemical receptors or materials.

The materials used can be modified with dopants, allowing facile tailoring of the sol-gel refractive index. Refractive index tuning is an advantage, not only for optimization of the coupler's waveguiding properties and refractive index sensitivity, but also allows the sensor to be tuned to have highest sensitivity in the refractive index ranges necessary for detection of protein/oligonucleotide-based molecular recognition (n=1.3-1.5), or for detection of other analytes.

Additional advantages of sol-gel materials over conventional semiconductor or inorganic crystalline materials include lower processing temperatures, as well as the unique ability to form uniform thin films.

### Brief Description of the Drawings

FIGURE 1 is a simplified diagram of a single planar coupler optical sensor according to the invention;
FIGURE 2 is a simplified drawing of a planar coupler array;
FIGURES 3A and 3B illustrate a waveguide and coupler geometry;
FIGURE 3C illustrates other potential waveguide and coupler geometries;
FIGURE 4 shows the intensity and phase distributions of the two modes within the coupling region;
FIGURE 5 is an end-on view of a coupler region enclosed in a fluidic channel including immobilized molecular receptors;
FIGURE 6 is a simplified schematic of a device with optical fiber inputs and a microfluidic channel in the top or "lid" of the device;
FIGURE 7 illustrates how surface silanol groups can be utilized for attachment of specified molecules (dopants) via hydrogen bonding, ionic bonding, or covalent bonding;
FIGURE 8 shows a thiol coupling chemistry applicable to the invention; and
FIGURE 9 shows a nitrene chemistry applicable to the invention.

### Detailed Description of the Invention

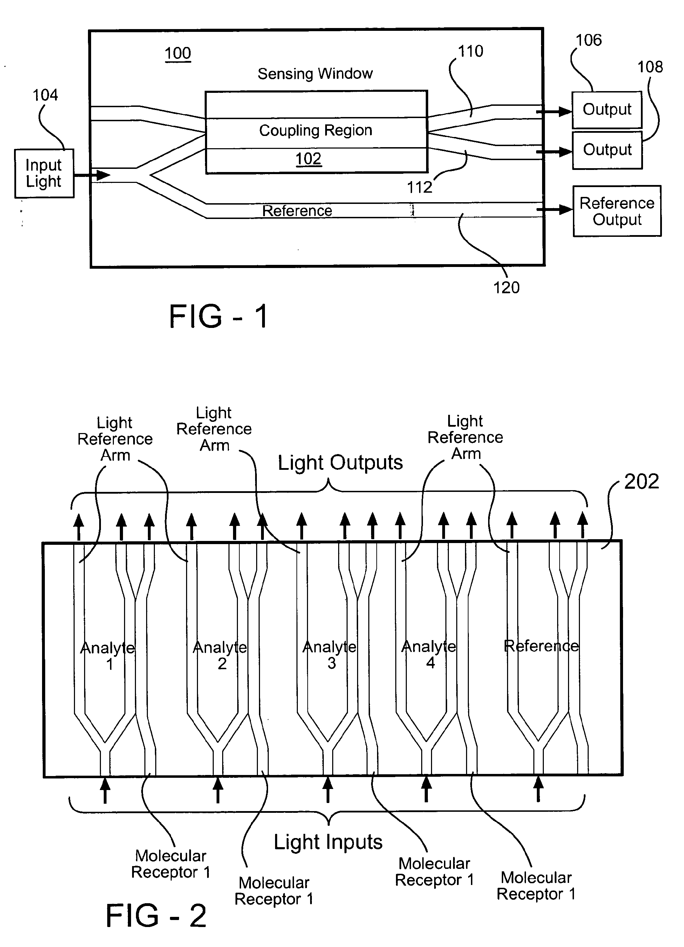

Figure 1 is a drawing that illustrates a sensor platform 100 according to the invention. The apparatus incorporates a dual-mode planar coupler 102 with a single optical input 104 and two outputs 106, 108. The coupled region 102 can support at least two electromagnetic modes, and the interaction of these fields determines the amount of light propagating down each of the output waveguides 110, 112. Changes in refractive index at the surface of the coupled region affect the interaction of the two modes and, therefore, the amount of light detected at each output 106, 108. The coupler also preferably includes a light intensity reference waveguide 120 as a means to measure and eliminate any remaining bulk index signal from the output signals.

To enhance throughput, the couplers are fabricated in an array format, as shown in Figure 2. In this way, numerous couplers will be modified with various molecular receptors with sensitivity to different analytes. A reference coupler 202 is preferably included on-chip that will be passivated or "blocked" with a protein, such as bovine serum albumin, or other bio- or non-biopolymer which has no specific interactions with the analytes. This reference allows for correction of non-specific interaction and bulk refractive index changes.

As discussed in the Summary of the Invention, the waveguides are preferably fabricated using sol-gel/organic polymer composite materials. Potential sol-gel formulations include photopolymerizable organic polymer substituents as well as non-silicate metal alkoxides. The waveguide structure will be fabricated using standard, well-understood semiconductor processing techniques, such as reactive-ion etching (RIE) and direct ultraviolet (UV) contact printing. The utility of the different fabrication methods will depend upon the chemical composition of the sol-gel materials selected and the geometry of the design.

Sol-gel materials have been used as waveguide materials for over 10 years and show promise as optical devices. These types of materials lend themselves well to modifications via heavy metal dopants, as well as covalent attachments to organic polymers. Addition of dopants is made to increase the refractive index of the sol-gel material above that of silica (n ≅ 1.45 in the visible and near IR). The refractive index of the hybrid organic-inorganic sol-gel films are chemically and photochemically tunable from 1.45-1.80. Since it is currently impossible to produce thick sol-gel films (1-10 µm) in a single coating unless dopants are added to increase the viscosity of the precursor solution, dopants play a crucial role in the material and optical properties of the waveguide.

One advantage of using sol-gel materials as a waveguide rather than conventional semiconductor or inorganic crystalline materials is the lower temperatures required for preparation. The special ability of sol-gels to form uniform thin films allows waveguide features to be fabricated with very little processing, resulting in inexpensive fabrication. Incorporation of organic copolymers into the silica sol-gel may override some of the disadvantages usually associated with the sol-gel glasses. For example, inclusion of the organic polymers may eliminate shrinkage of features during processing and fill residual fine pores. Another common disadvantage of sol-gel glasses, residual hydroxyls, becomes advantageous since hydroxyls are primary sites for covalent linkage of proteins and oligonucleotides.

Disadvantages of other lithography materials such as semiconductor (i.e. InGaAsP/InP) and inorganic crystal (i.e. Ti:LiNbO) include limits in ultimate thickness, complicated apparatus, time consuming processing, and high temperature during processing (>1000°C). Furthermore, differences in refractive index from semiconductor to optical fiber are significant (from 3 to 1.5 respectively), yielding optical connections with high loss.

The fabrication of sol-gel waveguides may take advantage of mature photolithographic techniques. Patterning of the waveguide features constructed from a sol-gel has primarily been done via light initiated polymerization using various photolithography techniques. Those techniques used to date are holographic interference, electron-beam, contact printing UV-illumination and projection photolithography. Sol-gel glass waveguides produced by UV-illumination have been fabricated as optical amplifiers, couplers, splitters, multiplexers and integrated gratings. An evanescent field overlap coupler has been constructed in the planar format from a UV patterned sol-gel material. Multiple splitters have been constructed in the same fashion. Sol-gel waveguides have been shown to function as chemical sensors but to date no work has appeared where they function as a biosensor.

The response of many sensors and assays currently in use is based on absorbance or fluorescence of the analyte molecule or of a dye used as a tag. Fluorescence is a much more sensitive technique than absorbance and, therefore, provides lower limits of detection. Numerous fluorescence-based sensors have been developed and many are in a planar or chip format. Unfortunately, most common analyte molecules are not natively fluorescent. Therefore, a fluorescent dye molecule or tag, such as a quantum dot, must be incorporated in the analysis. Methodologies include, for example, linking the dye/tag to the analyte molecule or the receptor, performing a "sandwich" assay with a secondary labeled molecular recognition element, or displacing fluorescently labeled molecules with the unlabelled analyte molecules in a competitive format.

Such techniques complicate detection by adding steps and reagents. In addition, the attachment of fluorescent tags can interfere with the molecular binding event. The typical protein limit of detection for fluorescence-based techniques is 1 ng/mL (5 pM for 150 kDa protein). In contrast, the invention preferably uses "label-less" detection methods to simplify detection by reducing sample preparation and eliminating reagents. Current state-of-the-art, label-less detection methods measure refractive index changes induced by analyte binding. Such techniques include surface plasmon resonance (SPR), resonant mirrors, and various interferometric methods.

As discussed above, sol-gel formulations are chosen to optimize physical properties, suitability for lithography, optical properties, and availability of functional groups for immobilization of receptors or other materials. Potential formulations include photopolymerizable organic polymer substituents as well as non-silicate metal alkoxides.

The most common method used to produce polymeric silica supports is the sol-gel process by which a solution of an alkoxysilane monomer is hydrolyzed and condensed into a silicate polymer. For example, an ethanol solution of tetraethoxysilane, in the presence of water and catalyst, undergoes partial hydrolysis and a condensation reaction to form a sol (a colloidal dispersion of particles in liquid). Depending on the reaction conditions and final processing, a number of different materials can be made. As the process of polymerization continues, a solid silicate network separates out of the solution (gel point). An alternate route to get to the gel point involves solution enrichment via loss of solvent. This route is the basis for application of silicate coatings to a variety of substrates by spin- or dip-coating methods. A heat treatment is applied to the final coating to ensure complete polymerization.

The coatings obtained by these methods are typically referred to as xerogel silicate materials and typically contain the desired silanol species which are necessary for the biolinker attachment step. It is worth noting that other metal oxides may be formed by similar methods. Incorporation of these alternative oxides (for example TiO₂ or V₂O₅) into the silicate materials results in complex coatings which have semiconductor properties which may be important for monitoring electrical changes in the sensor binding sites. Incorporation of additional metal oxides is also the key to raising the refractive index to a useable and tunable value as silicates typically have a refractive index value of less than n=1.43.

A disadvantage of applying coatings by the sol-gel methodology described above is that the compatible solvent systems consist of organic solvents. As coating thicknesses are driven by solution viscosity, the thin viscosity of organic solutions lead to coatings which are much too thin to provide the size features needed for the proposed waveguides. Using a laminate approach to build up to a thicker waveguide is difficult due to the enormous amount of time required to coat, cure and recoat until a desirable thickness has been obtained.

A solution is to incorporate a compatible polymer system which will thicken the coating solution. The ideal system will not only covalently link to the silicate polymer, but will also be photopolymerizable. The photopolymerizability provides the means to form the waveguide structure. Photolithography may be used to polymerize the waveguide features by copolymerizing the silicate organic copolymer system into a insoluble mass from which solvent washes are used to wash away the non-exposed (and thus non-copolymerized) areas. The choice of photocrosslinkable copolymer must also be made with consideration of the effect it will have on the refractive index.

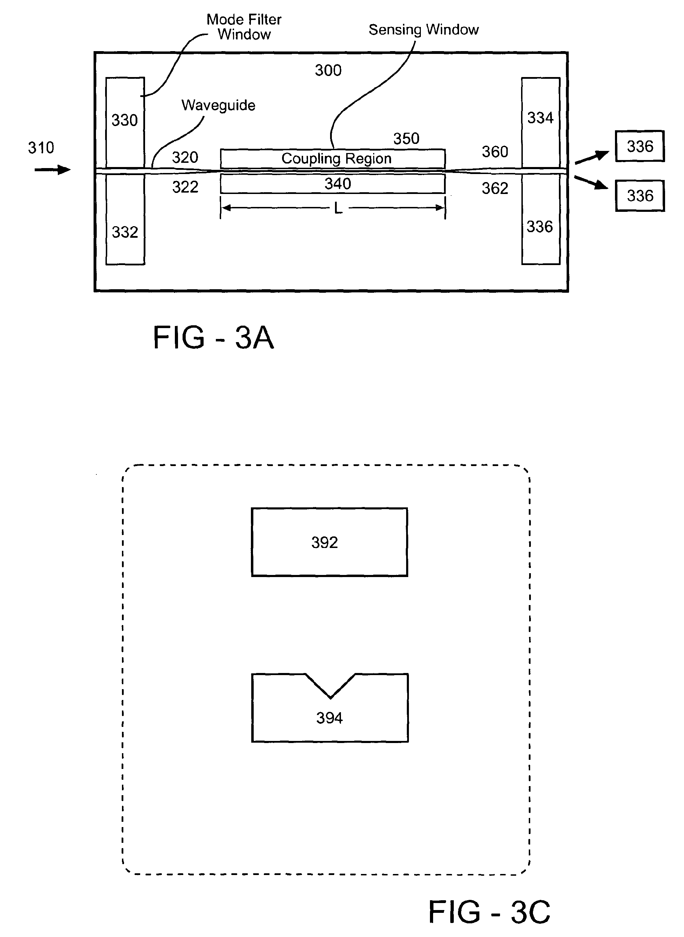

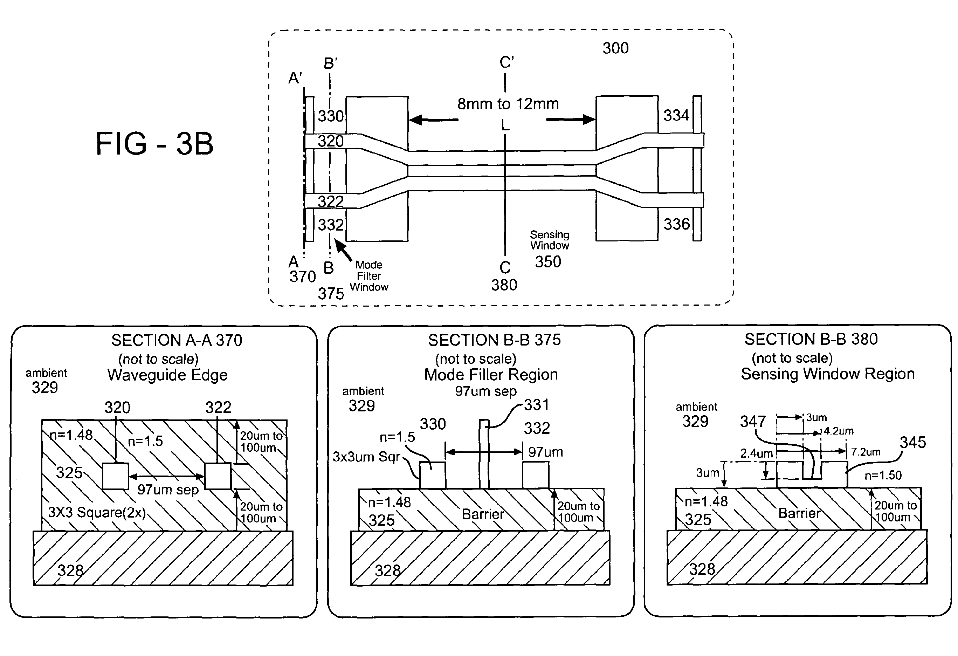

The core of the coupler device is the waveguide itself. An example of a design with preliminary dimensions for the waveguide portion of a single coupler is shown in Figures 3A and 3B. The device has a coupling or sensing region which supports two simultaneously propagating guided modes. The interaction (i.e. the coherent superposition) between these two modes, each with a different propagation constant, enables the coupling behavior. The mismatch in the speed of propagation between the two modes as they travel along the length of the coupling region causes the two modes to cycle in and out of phase with respect to each other, resulting in a periodic transfer of power from one side of the waveguide to the other. The propagation of the guided modes are each affected, although not equally, by the index of refraction within the clad or evanescent region. Intuitively, it may help to understand by picturing the evanescent 'tails' of each mode within the sensing region. The length of each tail is different and therefore each of the modes 'samples' the index in the evanescent or sensing region differently. The operation of the 'dual-mode' coupler as a sensor is based on the dependence of the propagation constants upon the index of refraction profile in the evanescent volume surrounding the coupling region.

All this discussion about evanescent fields should not be confused with the primary interaction mechanism between the two modes in the coupling region. Although the modes sample the sensing region via their respective evanescent fields, the primary or dominant coupling mechanism between the two modes is a direct one between the guided portions of the fields. This is an important distinction because there are devices that use evanescent coupling. In these cases, the two modes are separated by a region along the interaction or coupling length which has a lower refractive index (i.e. a clad region). For these devices the coupling between the two modes is weaker and therefore the interaction length is substantially longer than the device described within this invention.

The design of the coupling region in particular is critical to the coupler operation as a biosensor. This is illustrated in Figures 3A, 3B, and 3C. Figure 3A is a depiction of an overall top view of the planar biosensor chip, 300. In this example, the light, 310, enters from the left at one of the two input waveguides, 310. The light in the waveguide, 310, first passes through a region referred to as the mode filter window, 330. The purpose of this window, 330, is to strip or remove any cladding and high order modes that may have been excited by the input light launch conditions. The sensor operates via the interaction of the first two lowest order modes within the coupling region, 340. Any higher order or cladding modes would only add to spurious noise in the signal. It is therefore advantageous to eliminate these unwanted modes.

The mode filter window region, 330, can eliminate higher modes via a variety of methods. For example, the higher modes can be removed by introducing bends in the waveguide, 320. These bends would introduce higher loss in the higher modes as compared to the loss in the lowest mode. Given an adequate number of bends, the lowest order mode would solely survive. Tapering of the transverse dimension of the waveguide, 320, in this region would also eliminate the higher modes. The most flexible method may be the adjustment of the numerical aperture (NA) of the waveguide, 320, in this region. This can be done by varying the index of the cladding around the waveguide, 320. This can be accomplished by simply inserting a solution (a near-index matching fluid) with an easily variable index of refraction into the mode filter region, 330. This last method has been chosen for this design example. Additional mode filter windows, 332, 334, 336, are included to maintain the symmetry of the design and to filter the higher modes present within the output waveguides 360 and 362. This will be described later.

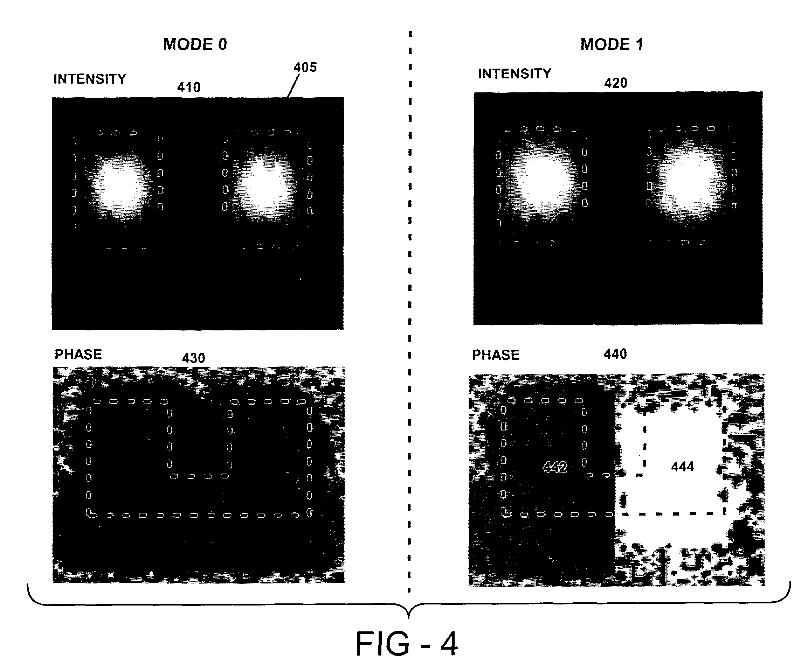

After passing through the mode filter window, 330, the light in the waveguide, 320, enters into the coupling region, 340. The lowest order mode of the light in the waveguide, 320, upon entering this coupling region, 340, ideally equally excites and fills the two lowest order modes of the core waveguide geometry in that region. Figure 4 illustrates the characteristics of these two modes of the waveguide at the entrance to this region. Both intensity and phase distributions are shown along with an overlay, 405, of the physical geometry of the cross section. The intensity distributions, 410 and 420, of the two modes are ideally essentially the same. In the plots 410 and 420, areas of high intensity are shown in white while low intensity is represented by black. The phase distributions, 430, 440, on the other hand, are not similar. The phase distribution, 430, of the zero mode is uniform, (i.e. it is zero across the entire cross section of the waveguide). This is represented by the mono-color within the overlay, 405, of the graph, 430. The phase distribution, 440, of mode one assumes two values (as indicated by the two different colored regions within overlay 405) as a function of position within the waveguide cross section. In region 442, the phase is zero as it is in the phase distribution of mode zero, 430. But in region 444, the optical field is 180 degrees out of phase with respect to region 442 and therefore also 430. These characteristics of the modes are essential to the proper operation of the device. The equal intensity distributions of the two modes allow for complete destructive and constructive interference between the two modes. The 180 degree phase change on one side of one of the modes allows for complete power to reside in either one side or the other of the waveguide geometry in this region. Lastly, the difference in the speed of propagation between the two modes allows for the periodic transfer of power from one side of the waveguide to the other as a function of the coupling length, L. The periodic transfer of power also ensures that there are locations at which there is an even split (i.e. 50% / 50%) in power along the length of the coupling region, 340.

The coupling region, 340, also coincides with the sensing window, 350. The sensing window region is the location for the molecular receptors to be used for the purpose of detecting a binding event in the presence of analyte. This binding event changes the refractive index in the region immediately surrounding the core waveguide structure of the coupling region, 340. As previously described, a change in the index in this region, 340, will affect the speed of propagation of the two modes of interest. If the length, L, of this coupling and sensing window region is chosen such that before a binding event occurs, the power split at the entrance to the output waveguides, 360, 362, is even (i.e. 50% / 50%), then a change in the refractive index upon the binding event will change the power split or ratio of the light in the output waveguides, 360 and 362. The length, L, can be chosen such that in the nominal condition (i.e. before binding), the end of the coupling region can be at any periodic 50% / 50% power transfer point. It has been found that the third period of this 50% / 50% point, (i.e. the third cross-over), is an optimum with regard to sufficient sensitivity to the change in index along with the suppression of changes due to noise sources.

Upon leaving the coupling region, 340, the light enters the output waveguides, 360 and 362. The optical power split is determined by the length of the coupling region and the index in the sensing region. The light in these waveguides then passes through mode filter windows, 334 and 336. These filters are essential in removing higher order modes and cladding modes and operate as previously described. These higher modes may have been introduced to the waveguide from light interacting in the coupling region, 340. Finally, the light in waveguides, 334 and 336, exits the chip, 300 and is directed towards detectors, 370 and 372. The ratio of the power signal from the two outputs can then be analyzed.

Figure 3B is a more detailed close up of the critical waveguide features of the design example. It depicts the cross section of the waveguide at various locations on the chip, 300. The dimensions, as indicated, were determined using commercially available waveguide design software (TempSelene by Alcatel Optronics Netherlands) to yield the desired waveguide performance. The intensity and phase profiles of Figure 4 were generated from the waveguide design as specified in Figure 3B by using this commercial software. For clarity, Figure 3B is not to scale.

Section A-A', 370, of Figure 3B depicts the cross section of the entrance (light launch side) to the chip, 300. Two waveguides, 320 and 322, are shown separated by a distance such that the guided modes within the waveguides do not interact. A distance of 97 um has been chosen for this case. Note also, because of symmetry, the exit of the chip, 300, is the same as section A-A', 370. At the exit, the proper choice of separation of the two waveguides ensures ease of separately detecting the light emitted from these two waveguides. The waveguides, 320 and 322, of section A-A' are surrounded by a clad material, 325. The refractive index of the clad material must be lower than the index of the core material which makes up the waveguides, 320 and 322. In this case, the following values were chosen: core index of 1.50; clad index of 1.48. It is important that the index difference between the core and clad along with the waveguide dimensions be maintained in order to assure the mode behavior of the waveguide as illustrated in Figure 4. The thickness of the clad material, 325, both above and below the waveguides must be sufficient such that the ambient, 329, and substrate, 328, materials do not affect the mode guiding capabilities of the waveguides. In this case, a clad thickness of 20 um to 100 um is chosen to be sufficient.

Section B-B', 375, depicts a cross section of the chip, 300, in the center of the mode filter window regions 330 and 332. In these regions, the waveguides, 320 and 322, are of the ribbed or raised type. The ambient layer, 329, surrounds three sides of the waveguides, 320 and 322. A solution or fluid of a selected or adjustable refractive index is chosen for the ambient medium in order to achieve the desired filtering of the higher order and cladding modes. The two mode filter window regions, 330 and 332, are separated by a barrier, 331, that is impermeable to the solution. In this way, the mode filtering capabilities of the two window regions can be individually adjusted. Because of symmetry, section B-B', 375, also represents the same configuration of the output mode filter windows, 334 and 336. The independent control of the mode filtering in the two separate windows as enabled by the barrier becomes more important on the output because both output waveguides nominally carry optical power. The waveguides, 320 and 322, in section B-B', 375, sit on top of a clad layer, 325, and a substrate, 328, as described in the previous cross section (A-A', 370). The clad to core index difference is also maintained at (ncore - nclad) = 0.02.

The two waveguides, 320 and 322, converge and join in the sensing window region, 350. Section C-C', 380, depicts a cross section of the center of that region and displays the dimensions critical to the proper operation of the coupling region. The general cross sectional geometry or shape, 345, of the core material in the coupling section is a rectangle with a notch, 347, in the top. The width and depth of the notch, 347, are critical for the determination of the mode behavior in the coupling region as illustrated in Figure 4. Figure 3C illustrates alternative shapes, 392, 394 for the waveguide in this region that may be used to yield similar or equivalent mode profiles as outlined in Figure 4.

The length, L, of the region, 350, is shown to be in the range from 8 mm to 12 mm. This spans an operating regime at the output from the second power cross-over to the fourth power cross-over under nominal conditions. This has been described previously. The ambient index is determined by the choice of buffer solution introduced into the sensing region, 350. The clad, 325, and the substrate, 328, layers beneath the waveguide, 345, are as described in section B-B', 385.

The waveguide structure is preferably fabricated using reactive-ion etching (RIE) or direct ultraviolet (UV) contact printing. The utility of the different fabrication methods will depend upon the chemical composition of the sol-gel materials selected and the geometry of the design. RIE of sol-gel films has been demonstrated successfully, so either lift-off definition of waveguide structures, or positive definition followed by etching, are available. The nature of sol-gel precursors makes lift-off of a thick photoresist (such as AZP 4620) an attractive option. UV contact printing is also an established sol-gel waveguide fabrication technique. Introduction of photoactive polymers into the sol-gel, will allow the waveguide structures to be defined by direct UV contact printing of the sol-gel film without the use of a photoresist.

As described above, the initial coupler cross-section will be rectangular or rectangular with a slot or groove etched along the length of the waveguide. Various fabrication techniques can be used to produce a groove with the desired geometry after the waveguide is formed by dip or spin-coating. To create a rectangular grove, the wafer may be planarized and lithographically patterned in order to etch a rectangular slot or groove into the coupler waveguide. Electron-beam lithography (EBL) could be utilized to create very small (0.1 micron) rectangular grooves. If an angular "V"-shaped groove would enhance performance, then this slot can be fabricated by focused-ion beam (FIB) lithography. Both of these processes have the advantages of not requiring planarization, and of being programmable - so that different groove widths, depths and even profiles may be obtained. If groove sizes greater than 1 micron in width are acceptable, then conventional photolithography is both a faster and cheaper option

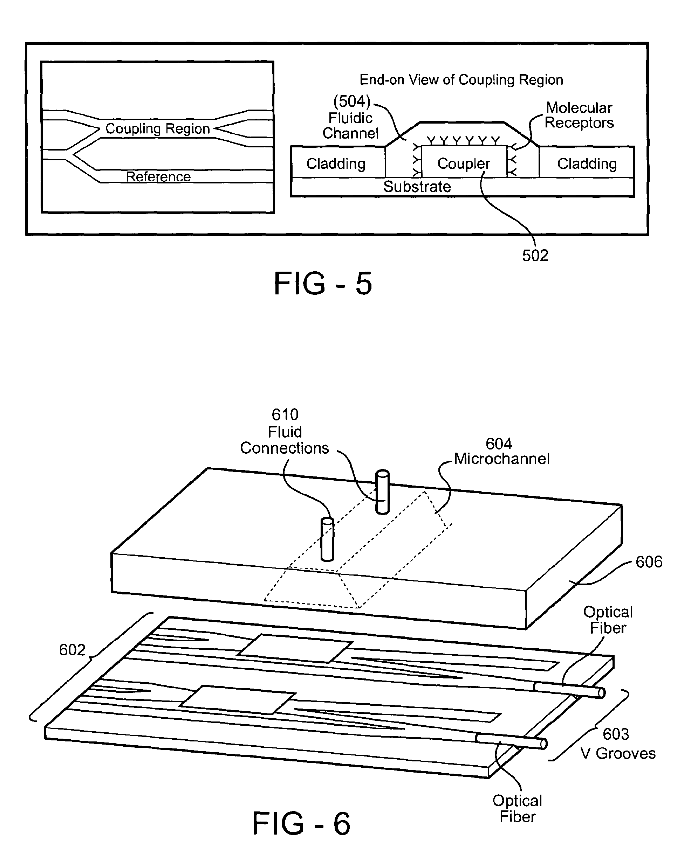

Figure 5 shows an end-on view of the coupling region 502 enclosed in a fluidic channel (506). Figure 6 is a simplified schematic of a device with optical fiber inputs 602 and a microfluidic channel 604 in the top or "lid" 606 of the device. This testing platform includes the waveguide devices, sample fluid delivery through connections 610, light inputs and outputs 602,603, detectors (not shown) and data collection (not shown). Although a single fluid channel is shown for simplicity, multiple, parallel channels may also be provided, preferably parallel to the waveguides.

The molecular receptors are immobilized on the waveguide surface in the coupling region. Adsorption of molecular receptors on a surface is a straightforward derivatization method that is often used in conjunction with immunoassays and sensors. This technique has been used to immobilize receptors on a variety of substrates including glass, quartz, and plastics. The simplicity of adsorption makes it especially well-suited to preliminary experiments and single-use devices.

Covalent attachment of molecular receptors has several advantages over simple adsorption; for one, it offers the possibility of regenerating the sensor. For example, an antigen can be denatured from a sensing antibody by changing the elution buffer pH. Other advantages include proper orientation of the receptor, less denaturation of the receptor, and less leaching or loss of receptor over time.

When covalently linking biomolecules to a solid surface, it is important to consider the method of attachment. The method must be mild enough, so as not to denature or alter the activity of the receptor, and it must link the receptor in an orientation that keeps the active site available for binding.

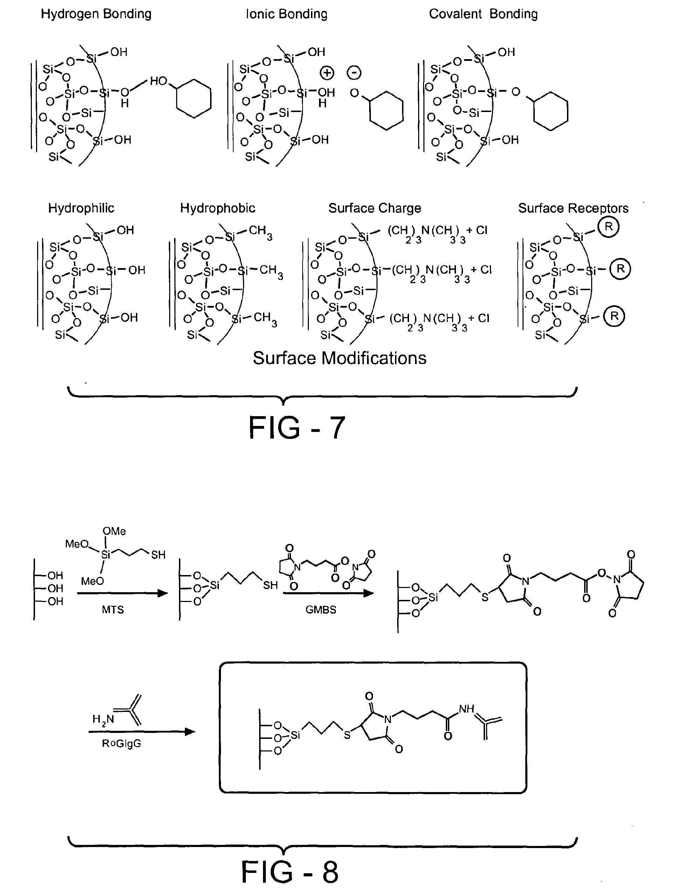

One method utilized to derivatize silicates is doping. Doping refers to the process of adding substances such as chemical reagents or bio-receptors to the matrix. The surface of the silica is covered with silanol (SiOH) groups. The surface silanol groups can be utilized for attachment of specified molecules (dopants) via hydrogen bonding, ionic bonding, or covalent bonding, as illustrated in Figure 7. It is the chemical versatility of the silanol group that makes silicates attractive for doping applications.

One strategy for covalently linking the molecular receptors to SiOH groups, modeled after the method of Ligler, as taught in U.S. Patent No. 5,077,210 and incorporated herein by reference, is shown in Figure 8. The waveguide surface silanated with 3-mercaptopropyl-trimethoxysilane (MTS). The free thiol group is then reacted with N-[γ-maleimidobutyryloxy]-succinimide ester (GMBS). The GMBS acts as the linker between the activated surface and the reactive amino groups on molecular receptors such as antibodies. In order to use this linking chemistry for oligonucleotides, 5' amino-modified oligonucleotides will be purchased. Couplers utilized as reference couplers will preferably be passivated or "blocked" with bovine serum albumin (BSA) or an appropriate mixture of unreactive proteins or polymers.

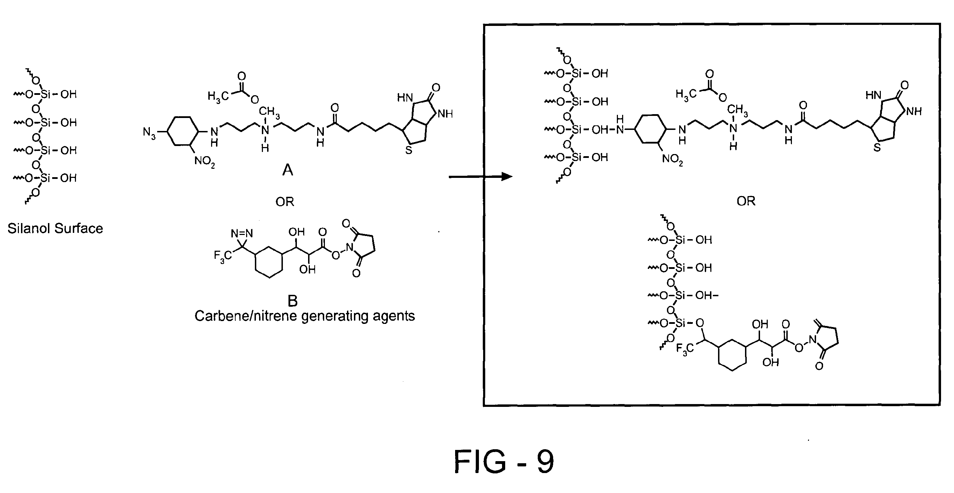

Additional coupling chemistry techniques may also be utilized. For example, UV illumination of a (trifluoromethyl) diazarine or a ntiroaryl azide can be used to produce a carbene (C:) or a nitrene, respectively (Figure 9). These moieties react readily with nucleophiles and have been used to derivatize a variety of substrates, including glass, quartz and plastics. Therefore, this technique provides a facile method for derivatization of waveguides fabricated from hybrid organic-inorganic sol-gel materials. The photoactive (nitrene/carbene - generating) group is attached to a linker group, such as a biotin or a succinimidyl ester. When "photobiotin" compounds are used, a streptavidin is used to link biotinylated receptors to the surface. When the succinimidyl ester is used biomolecules are linked through their amino groups, as described above. Other materials, including polymers may be applied to the coupling region. Such films may have specific or non-specific interactions with analyte molecules.

In the basic coupler design the signals are generated by monitoring the optical power at the output fibers of the couplers. The change in the power ratio between the two outputs is a direct measure of the change in the coupling ratio between the two output fibers. This, in turn, is interpreted as a change in the refractive index in the coupling region of the waveguide. The sensitivity in determining changes in the power ratio of the output signal is directly proportional to the sensitivity in determining index changes. This is essentially a DC measurement. A common technique to increase detection sensitivity is to convert from a DC measurement to an AC measurement (i.e. modulate the signal). Depending on the modulation technique used, a serious impact on the waveguide geometry and electro-optical interfaces may occur. Nevertheless, the gain in sensitivity may outweigh these implementation disadvantages.

Several signal modulation options may be utilized according to the invention. One simple technique involves modulating the amplitude of the light source such as with a chopper. This would allow locking onto the output signal and thus eliminate some of the noise sources. There would be minimum change to the system to employ this technique.

A more sensitive signal extraction technique could be used if the change in index could be converted to a change of phase between two modulated waveforms (a reference and a measurement signal). This is commonly done in interferometric instruments, where the electrical phase difference between a reference and a test signal is directly proportional to the optical phase difference in the reference and test paths of the interferometer. In interferometry, the signal can then be modulated by introducing phase modulation in each of the paths or, alternatively, by inserting two wavelengths (one for each path) and monitoring the beat frequency between the wavelengths. In either case, the measurement turns into an electrical signal phase measurement between the two waveforms. This is advantageous because the information is carried on AC waveforms, or carriers, rather than in DC form and extraneous noise can be eliminated.

Similarly, the same advantages may be realized by converting the coupler intensity measurement into a phase measurement. Applicable techniques include phase modulation of a test coupler with respect to a reference coupler or by introducing a variable wavelength light source. The coupling of light into the output waveguides for the planar waveguide has a periodic behavior with respect to at least 3 factors; index in the coupling region, length of the coupling region, and the wavelength of the light. Modulation of the signal may be achieved by varying any of these factors. Modulation of the length appears to be impractical, but both the modulation of the wavelength or the index appear feasible.

Modulation of the wavelength is appealing because this technique does not require changes to the geometry of the waveguide. However, the magnitude of the required wavelength range and the magnitude of the ramp time determine the modulation frequency. In order to achieve high modulation frequencies, the length of the waveguide coupling region may be increased. This would also increase the sensitivity of the device to unwanted factors such as temperature. This is an example of the trade-off between increasing sensitivity to wavelength and waveguide parameters.

The modulation of the index could be achieved by using an electro-optic (ferroelectric) material. Waveguide Mach-Zehnder interferometers using non-linear crystalline materials such as lithium niobate (LiNbO₃), or ZnO have been developed. These devices modulate the index by the application of a voltage across the crystal. Modulation rates for these devices can be very fast (> 1Mhz) and the detection electronics would have to become more sophisticated.

It should be noted that although specific reference is made herein to the "analyte binding of molecular receptors," the invention is not limited in this regard and is applicable to any type of organic/inorganic material, so long as the interaction of one component causes a change in any optical property detectable by the apparatus. Accordingly, the invention is applicable to any chemical/biochemical/bioaffinity/ immuno-type interactions of ligands or other types of respective binding partners. Examples include, but are not limited to, bio- and non-biopolymers, antigen-antibody, carbohydrate-lectin, receptor-ligand, binding protein-toxin, substrate-enzyme, effector-enzyme, inhibitor-enzyme, complimentary nucleic acid strands, binding protein-vitamin, binding protein-nucleic acid, reactive dye-protein, and reactive dye-nucleic acid interactions.

## Claims

1. A planar optical sensor, comprising:
at least one input waveguide and at least two output waveguides in optical communication with a coupling region having a sensor surface configured to receive a multiplicity of immobilized chemical or biological receptors thereon;
a source of light directed to the input waveguide;
the coupling region supporting at least two electromagnetic modes of propagation such that chemical or biological binding to the receptors causes a change in the refractive index near the surface of the waveguide of the coupled region affecting the interaction of the propagation modes; and
a detector for detecting differing aspects of the light propagating down each of the output waveguides.

2. The planar optical sensor of claim 1, further including a light intensity reference waveguide to measure and eliminate bulk index signals from the output signals.

3. The planar optical sensor of claim 1, including a plurality of coupling regions, each with input and output waveguides, fabricated in an array format.

4. The planar optical sensor of claim 3, including a reference coupler passivated with a substance with no specific interactions with the analytes.

5. The planar optical sensor of claim 4, wherein the substance is the protein bovine serum albumin or some other bio- or non-biopolymer.

6. The planar optical sensor of claim 1, wherein the waveguides, coupling region, or both, are fabricated with a sol-gel material.

7. The planar optical sensor of claim 6, wherein the sol-gel is fabricated from a sol-gel/organic polymer composite material.

8. The planar optical sensor of claim 7, wherein the sol-gel/organic polymer composite includes a photopolymerizable organic polymer substituent.

9. The planar optical sensor of claim 7, wherein the sol-gel/organic polymer composite includes a non-silicate metal alkoxide.

10. The planar optical sensor of claim 7, wherein the sol-gel is photopolymerized or modified with a dopant to tailor the refractive index.

11. The planar optical sensor of claim 10, wherein the refractive index is tuned for protein/oligonucleotide-based molecular recognition.

12. The planar optical sensor of claim 1, including the use of a label-less detection method to measure refractive index changes induced by the binding.

13. The planar optical sensor of claim 12, wherein the label-less detection method includes an interferometric technique.

14. The planar optical sensor of claim 1, wherein the detector is operative to measure a change in the coupling ratio between the two output waveguide signals.

15. The planar optical sensor of claim 1, wherein a differing aspect of the light propagating down each of the output waveguides includes the difference in phase.

16. The planar optical sensor of claim 15, wherein the difference in phase is derived from the phase modulation of a test coupler with respect to a reference coupler.

17. The planar optical sensor of claim 15, wherein the difference in phase is derived by introducing a variable wavelength light source.

18. The planar optical sensor of claim 15, wherein the light coupled into one or both of the output waveguides is modulated.

19. The planar optical sensor of claim 18, wherein the modulation is a function of wavelength.

20. The planar optical sensor of claim 18, wherein the modulation is a function of the index in the coupling region.

21. The planar optical sensor of claim 18, wherein the modulation is a function of the length of the coupling region.

22. The planar optical sensor of claim 1, wherein the chemical or biological binding results from one of the following processes:
bio- and non-biopolymers, antigen-antibody, carbohydrate-lectin, receptor-ligand, binding protein-toxin, substrate-enzyme, effector-enzyme, inhibitor-enzyme, complimentary nucleic acid strands, binding protein-vitamin, binding protein-nucleic acid, reactive dye-protein, and reactive dye-nucleic acid.

23. The planar optical sensor of claim 1, wherein at least two of the modes exhibit a different propagation constant causing a periodic transfer of power from one side of the waveguide to the other.

24. The planar optical sensor of claim 1, further including a mode filtering window associated with input waveguide to remove any cladding and high order modes.

25. The planar optical sensor of claim 1, further including a mode filtering window associated with the output waveguides to maintain symmetry or filter higher modes.

26. The planar optical sensor of claim 1, wherein the coupling region has a length such that in the nominal condition before binding the end of the coupling region is at a periodic 50:50 power transfer point.

27. A planar optical sensor, comprising:
one or more input optical waveguides, each having a core, the cores being coupled for exchange of optical signals in a coupling region;
a plurality of output optical waveguides emerging from the coupling region;
the coupling region distributing an input optical signal incident to one of the input optical waveguides between the plurality of output optical waveguides;
a first immunoreactant coated on the coupling region, the first immunoreactant being capable of specifically binding to a target analyte;
a source injecting light into at least one of the input optical waveguides, whereby an evanescent region is produced surrounding the coupling region; and
apparatus for measuring and comparing the magnitude of the light emitted by the output optical waveguides.

28. The planar optical sensor of claim 27, wherein the first immunoreactant is an antibody or an antigen.
